# EUROPEAN PATENT SPECIFICATION

(11) **EP 1 181 046 B1**
(45) Date of publication and mention of the grant of the patent: **27.08.2003**
(21) Application number: 00945698.9
(22) Date of filing: 02.06.2000
(51) Int. Cl.: A61K 38/44, A61P 35/00, C12N 9/02

(54) **10-FORMYLTETRAHYDROFOLATE DEHYDROGENASE AS THERAPEUTICAL AGENT**
10-FORMYLTETRAHYDROFOLATE DEHYDROGENASE ALS THERAPEUTISCHEN MITTEL
10-FORMYLTETRAHYDROFOLATE DESHYDROGENASE EN TANT QU'AGENT THERAPEUTIQUE

(30) Priority: 03.06.1999 IT MI991243; 20.10.1999 IT MI992197
(43) Date of publication of application: 27.02.2002
(73) Proprietor: Pharmaproducts UK Limited, Liverpool, Merseyside L2 9TL (GB)
(72) Inventor: BARTORELLI, Alberto, 3963 Crans-sur-Sierre (CH)
(74) Representative: Minoja, Fabrizio, Dr.
(86) International application number: EP0005066
(87) International publication number: WO00074711

(56) References cited:
- WO-A-00/73330
- R.J. COOK ET AL.: "ISOLATION AND CHARACTERIZATION OF cDNA CLONES FOR RAT LIVER 10-FORMYLTETRAHYDROFOLATE DEHYDROGENASE" JOURNAL OF BIOLOGICAL CHEMISTRY, vol. 266, no. 8, 15 March 1991 (1991-03-15), pages 4965-4973, XP002157451 BALTIMORE, MD, US cited in the application

## Description

The present invention relates to the use of 10-formyltetrahydrofolate dehydrogenase as therapeutical agent, in particular as cytotoxic and antitumour agent.

10-Formyltetrahydrofolate dehydrogenase is an enzyme present in the liver and in the nervous system of mammals. No therapeutical use for such enzyme has been disclosed up to now.

cDNA from rat 10-formyltetrahydrofolate dehydrogenase has been disclosed in J. Biol. Chem. 266(8), 4965-4973, 1991, while cDNA of the same human enzyme has been disclosed more recently (Biochem. Mol. Biol. Int., 47(3), 407-415, 1999).

Furthermore, methods for the preparation of the recombinant enzyme are known from Protein Expression Purif. 6, 457-64, 1995 and Biochem. J. 306(3), 651-5, 1995.

WO 00/73330A, published on 7.12.2000, discloses the use of 10-formyltetrahydrofolate dehydrogenase for the treatment of a condition characterized by insulin resistance.

It has now been found that mammal 10-formyltetrahydrofolate dehydrogenase is capable of inducing a marked cytotoxic response against tumour cells, when administered to tumour-bearing patients or animals.

This cytotoxicity seems to be mediated by cytotoxic antibodies to human tumour cells, particularly carcinomas and adenocarcinomas.

Cytotoxicity can be quantified in vitro on Jurkat and Kato III cells using conventional methods, based for example on the use of commercial kits such as the CDC-UK kit (Pharmaproduct). In particular, the appearance of cytotoxicity in rabbits serum was observed already after a first treatment with the enzyme (1 mg/animal in saline solution) on Jurkat and Kato III cells.

Therefore, the invention also relates to pharmaceutical compositions containing as active ingredient an effective dose of 10-formyltetrahydrofolate dehydrogenase.

The compositions of the invention will be administered to tumour patients using the conventional administration routes for proteins and polypeptides, for example the subcutaneous or intramuscular routes. The treatment may be repeated, a treatment comprising one-two week separated administrations of doses ranging from 0.1 to 20 mg of enzyme being preferred.

Furthermore, it has surprisingly found that it is possible to induce high cytotoxicity by administering the enzyme even at very low dosages, such as 1.10⁻⁴ - 1.10⁻¹⁰ g, through the sublingual route, in the form of granules or drops of 1% water-alcoholic solutions or suspensions in ethanol, with concentrations of active ingredient ranging from 10⁻⁶ to 10 ⁻¹⁰ M.

10-Formyltetrahydrofolate dehydrogenase can be prepared by conventional recombinant DNA methods or it can be extracted from the liver of animals, for example from liver of bovine, ovine or swine. Goat liver proved to be a particularly abundant source of this enzyme.

The extraction process comprises the treatment of livers with solutions buffered at pH 7.4 (PBS) followed by precipitation with 15% polyethylene glycol 6000, chromatography on TSK-DEAE or DEAE-Sephacell at pH 8, elution with 0.3 M NaCl and purification on TSK SW3000.

The following example illustrates the invention in greater detail.

### EXAMPLE

### Extraction

50 g of goat liver are homogenized, suspended in 400 ml of PBS 0.01 M pH 7.2, stirred for 30 minutes at 4°C and centrifuged on JA14 at 14,000 RPM for 30 minutes. After that, the product is filtered with suction; then through 1.2 µm filter, finally through 0.45 µm filter.
Volume: 340 ml conc. 10.9 mg/ml.

### Fractional precipitation with PEG 6000.

336 ml of the above sample are treated with 5% powder PEG 6000 (16.8 g). The whole is stirred for 1 hour at 4°C, then centrifuged on J 6 at 4,000 g for 30'.

The pellet is taken up into 61 ml of 0.03M Tris/HCl pH 8, whereas the supernatant (340 ml) is reprecipitated with 5% PEG 6000 (17 g), then 10% PEG 6000 stirring for 1 hour at 4°C.

After centrifuging on J6 at 4,000 g for 30', the pellet is taken up with 62 ml of 0.03M Tris/HCl pH 8.

The supernatant (345 ml) is treated with 5% PEG 6000 (17,25 g), then again with 5% PEG 6000, stirring for 1 hour at 4°C, then centrifuged on J6 at 4,000 g for 30'.

The supernatant is discarded, and the pellet is taken up into 200 ml of 0.03M Tris/HCl pH 8.
5% PEG pellet volume: 61 ml, cone. 9.34 mg/ml.
10% PEG pellet volume: 62 ml, cone. 13 mg/ml.
15% PEG pellet volume: 200 ml, conc. 3.38 mg/ml.

### DEAE - Sephacell

About 150 ml of DEAE-S resin are equilibrated in 0.03 M Tris/HCl buffer pH 8. The resin is incubated with the 15% PEG sample for 30 minutes at room temperature + 200 ml of washing.

Leg 1: 200 ml 0.5M Tris/HCl pH 8 for 30 minutes at r.t. + 200 ml of washing.

Leg 2: 200 ml 0.03M Tris/HCl pH 8 + 0.3M NaCl for 30 minutes at r.t. + 200 ml of washing.

Leg 3: 200 ml 0.03M Tris/HCl pH 8 + 1M NaCl for 30 minutes at r.t. + 200 ml of washing.

The following samples are thereby obtained:
S.B. volume: about 400 ml conc: 294 γ/ml.
LEG 1 volume: about 400 ml conc: 1.14 mg/ml.
LEG 2 volume: about 400 ml conc: on PM 30.
LEG 3 volume: about 400 ml conc: 137 γ/ml.
LEG 2 is conc. on PM 30 to an about 20 ml final volume, concentration of about 3.6 mg/ml.

### SW3000 prep.

LEG 2 from DEAE-S obtained above is purified in prep. SW3000 prep. (10 runs, 2 ml each).

Four fractions are eluted, the second being concentrated on PM 30 and dyalised against H₂O to a final volume of about 2 ml, concentration of about 1.5 mg/ml.

## Claims

1. The use of 10-formyltetrahydrofolate dehydrogenase for the preparation of cytotoxic and antitumor medicaments.

## Patentansprüche

1. Verwendung von 10-Formyltetrahydrofolate-Dehydrogenase zur Herstellung von zytotoxischen und antitumoralen Arzneimitteln.

## Revendications

1. Utilisation de la 10-formyltétrahydrofolate déshydrogénase pour la préparation des médicaments cytotoxiques et antitumorals.
